(19) Europäisches Patentamt
European Patent Office
Office européen des brevets

(11) Publication number: **0 055 029**
**B1**

(12) **EUROPEAN PATENT SPECIFICATION**

(45) Date of publication of patent specification: **21.11.85**

(51) Int. Cl.$^4$: **A 61 K 9/06,** A 61 K 7/48, A 61 K 31/405

(21) Application number: **81305526.6**

(22) Date of filing: **24.11.81**

(54) **Preparations for the treatment of dermatoses.**

(30) Priority: **09.12.80 JP 173667/80**

(43) Date of publication of application:
**30.06.82 Bulletin 82/26**

(45) Publication of the grant of the patent:
**21.11.85 Bulletin 85/47**

(84) Designated Contracting States:
**BE DE FR GB IT NL SE**

(56) References cited:
**EP-A-0 036 138**
**DE-B-1 617 653**
**FR-M- 1 443**
**US-A-3 629 412**
**US-A-4 126 681**

**The file contains technical information submitted after the application was filed and not included in this specification**

(73) Proprietor: **KOWA COMPANY, LTD.**
**6-29, 3-chome Nishiki**
**Naka-ku Nagoya-shi Aichi-ken (JP)**

(72) Inventor: **Nagai, Hidetaka**
**1660-50, Takamine-cho**
**Hachiohji-shi Tokyo (JP)**
Inventor: **Muramatsu, Toyojiro**
**1-4-9, Sayamadai**
**Sayama-shi Saitama-Ken (JP)**
Inventor: **Inagi, Toshio**
**2927-2, Mihara-cho**
**Tokorozawa-shi Saitama-Ken (JP)**

(74) Representative: **Webb, Frederick Ronald et al**
**G.F. Redfern & Co. Marlborough Lodge 14**
**Farncombe Road**
**Worthing West Sussex BN11 2BT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 055 029 B1

**Description**

The present invention relates to preparations for the treatment of dermatoses, and more particularly to external preparations (i.e. preparations intended for external application) for the treatment of dermatoses which comprise indomethacin as an active component.

At present, indomethacin is being widely used as a non-steroidal, anti-inflammatory agent. However, medication with indomethacin induces adverse effects such as gastoenteric disorders in the form of stomach-ache and vomitting, due primarily to oral administration. This has given an impetus to the development of medicaments in which indomethacin is applicable in the form of a suppository, a combined drug, or a prolonged action drug.

A gelled ointment containing indomethacin has recently been developed for the treatment of rheumatism, arthritis, osteoarthritis, scaplulohumeral periarthritis, myalgia and like conditions in the field of orthopedic surgery.

Indomethacin exhibits an outstanding remedial effect on dermatoses, but a gelled ointment prepared from indomethacin is irritating when applied directly to injured skin. The topical application of indomethacin is practically impossible, and thus, indomethacin must be administered through an oral route or in the form of a suppository. The medication of indomethacin in such a form, however, involves the above-mentioned side effects and requires larger dosages so as to be rendered actually effective than does the external use. There has thus been a demand for the development of an external preparation for the treatment of dermatoses that can be applied directly to any affected parts of the skin. Such preparation has not hitherto been realized because indomethacin is neither well retained on the skin nor well absorbed by the skin and is likely to cause ulcers on injured skin.

In order to overcome the foregoing disadvantages of the existing techniques, the present inventors have carried out a series of researches leading to the development of external preparations of indomethacin for the treatment of dermatoses which can be applied directly to any affected skin parts.

Because of the insolubility in water and common solvent, indomethacin is merely suspended in a base when prepared as an ointment or cream, and the suspended component is not well absorbed by the skin and consequently does not exhibit any great remedial effect. As a result of further studies to solve this problem, the present inventors have found that it is possible to improve the properties of indomethacin as far as its retention on and absorption by the skin are concerned, and to improve its remedial effect, if a specific adjuvant is added to increase the release of indomethacin from the preparation.

It is accordingly an object of the present invention to provide an external preparation for the treatment of dermatoses which contains indomethacin and which is completely devoid of the disadvantages of the previously used techniques, and which can be applied directly to an affected skin part.

According to the invention, there is provided an external preparation in the form of an ungelled ointment or cream for the treatment of dermatoses, comprising as essential ingredients, other than are required to convert or maintain the preparation in ungelled ointment or cream form, or conventionally used in such ointments or creams, an effective amount of indomethacin and at least one adjuvant selected from a liquid monohydric alcohol, a polyhydric alcohol, an ester of a monobasic fatty acid and a surface active agent, but not including N,N-diethyl-m-toluamide, wherein said liquid monohydric alcohol is ethanol, isopropanol or benzyl alcohol, said polyhydric alcohol is propylene glycol, said ester of a monobasic fatty acid is isopropyl palmitate, lauryl lactate or decyl oleate, and said surface active agent is polyoxyethylene sorbitan monooleate, a polyoxyethylene hardened castor oil derivative or a polyoxyethylene higher alcohol ether.

The external preparations for the treatment of dermatoses according to the invention can be prepared by incorporating indomethacin with agitation into an externally useful common base, preferably together with the adjuvant or adjuvants for increasing the release of indomethacin.

Suitable bases for external use in the invention include those bases which do not irritate even injured parts of the skin and are employed generally for the preparation of ointments and creams.

In addition to the selected adjuvant or adjuvants, fatty acid diesters, such as diisopropyl adipate; essential oils such as menthol, and camphor; and crotamiton, may also be present in the preparation.

The external preparations for the treatment of dermatoses, according to the invention, preferably contain 0.1 to 7% by weight, more preferably 0.5 to 5.0% by weight of indomethacin as an active component and preferably from 0.1 to 50% by weight of one or more adjuvants for increasing the release of indomethacin. The external preparation may additionally contain antioxidants, surface active agents, antiseptics and pH-adjusting agents.

The external preparations according to the invention are particularly effective for the treatment of contact-type dermatitis, atopic dermatitis, psoriasis vulgaris, acute and chronic eczema, zoster, decubitus, solar dermatitis, radio-ulcers, burn ulcers and similar conditions.

In addition, the external preparations of the invention are stable in storage over a long period of time and are not irritating to the skin, and can thus be applied directly to any affected parts of the skin. As compared with conventional oral administration or suppository medication, the topical application of indomethacin as contemplated by the invention exhibits a superior anti-inflammatory analgesic action with a very small quantity of the active component.

German Auslegeschrift No. 1 617 653 in the name of Meiji Seika Kaisha Ltd., discloses indomethacin

preparations for external use in which indomethacin is dissolved in a base containing at least one diester of the formula:—

$$ROOC—(CH_2)_n—COOR$$

where R is an aliphatic alkyl group with 1 to 4 carbon atoms, and n is a whole number from 1 to 8. Such esters have a marked capacity for transporting indomethacin through the skin, when the preparations are applied externally. The preparations may be in the form of an ointment or cream.

European Patent Application A—0 036 138, in the name of Merck & Co. Inc., which has a date of filing prior to the date of filing of the present application, but was published after that date, and therefore falls under Article 54(3) of the European Patent Convention, describes and claims compositions of matter for topical application comprising a biologically effective amount of at least one bio-affecting agent and a skin permeation enhancing amount of N,N-diethyl-m-toluamide. Among the bio-affecting agents referred to in the description are non-steroidal anti-inflammatory agents, including indomethacin. Example XXII of this application describes the preparation of oil/water creams containing 1% by weight of indomethacin as the bio-affecting agent and 5% by weight of N,N-diethyl-m-toluamide.

The invention will now be further described with reference to the following experimental examples and inventive examples which are provided for purposes of illustration only and are not intended to be limiting unless otherwise specified.

Reference will also be made to the drawing which is a graph showing the relationship between the amount of indomethacin released and time for preparations according to certain of the inventive examples and for a control preparation.

Experimental Example 1
Inhibitory effect on carrageenan-induced edema

Wister male rats each weighing about 200 g, arranged in groups each consisting of twelve animals, were given subcutaneously 0.05 ml of a 1% carrageenan solution on their hind right paws. Immediately thereafter, about 100 mg of an ointment prepared as in Example 1 below, was coated onto each injected region which was covered with a commercially available plastic film [Kure-Wrap (Trade Mark) made by Kureha Chemical Co., Ltd.] and held thereon by means of gauze. Two hours later, the film and gauze were removed. One hour after such removal, the weight of edema was measured. A control group of animals was coated only with an ointment base and thereafter treated in the same manner as in the subject test groups.

The results obtained are as shown in Table 1:—

TABLE 1

| Agent | Weight of edema (g) mean±error | Inhibition ratio (%) |
|---|---|---|
| Control | 0.73±0.04 | — |
| Indomethacin ointment (1%) | 0.57±0.03 | 21.9* |

*$p<0.01$

Experimental Example 2
Inhibitory effect on acceleration of blood vessel permeability

Guinea pigs arranged in groups each consisting of six animals were coated twice with about 50 mg of the ointment prepared as in Example 1 below, on the skin of their backs after the hair had been removed, at an interval of one hour. One hour after the second coating, a 1% Evans Blue solution was injected intravenously, and 10 μg of a histamine hydrochloride solution was immediately injected intradermally into each ointment-coated region. 30 minutes later, the animals were decapitated and exsanguinated. Each blue-dyed skin was exfoliated, and the pigment was extracted with pyridine and determined. A control group of animals was coated only with an ointment base and thereafter treated in the same manner as in the subject test groups.

The results obtained are as shown in Table 2.

TABLE 2

| Agent | Evans Blue (μg/region) mean±error | Inhibition ratio (%) |
|---|---|---|
| Control | 49.2±5.3 | — |
| Indomethacin ointment (1%) | 33.1±4.9 | 32.7** |

$**p<0.05$

Experimental Example 3
Release from various bases

Ointments prepared respectively as in Examples 3 to 8 below, each weighing 2 g, were placed in glass tubes each having a cellulose membrane adhering to one side of the tube. The tube was dipped in 30 ml of a phosphate buffer solution (pH 7.0) and then shaken at 25°C. After an interval of 1, 3, 5 and 10 hours, sampling was made to determine the quantities of indomethacin dissolved. For purposes of control, the steps noted above were carried out for an ointment of the same recipe as in Example 8, except that no adjuvant was added for increasing the release of indomethacin.

The results obtained are shown in the accompanying drawing in which curve 1 represents the ointment prepared as in Example 3; curve 2, Example 4; curve 3, Example 5; curve 4, Example 6; curve 5, Example 7; and curve 6, the control.

Example 1

Materials

| | |
|---|---|
| 1) Indomethacin | 1.0% by weight |
| 2) White Vaseline (Trade Mark) | 15.0% by weight |
| 3) Liquid paraffin | 6.0% by weight |
| 4) Cetanol | 3.0% by weight |
| 5) Stearyl alcohol | 3.0% by weight |
| 6) Polyoxyethylene cetyl ether | 2.5% by weight |
| 7) Propylene glycol | 5.0% by weight |
| 8) Purified water | An amount sufficient to bring the final weight to 100% |

Method

A) Heat and melt 1) to 7).

B) Add heated 8) to the mixture of 1) to 7).

C) Stir the resulting mixture until it has cooled to room temperature and becomes a semi-solid preparation.

Example 2

Materials

| | |
|---|---|
| 1) Indomethacin | 3.0% by weight |
| 2) Benzyl alcohol | 10.0% by weight |
| 3) Sorbitan sesquioleate | 1.5% by weight |
| 4) Liquid paraffin | 12.0% by weight |
| 5) Solid paraffin | 6.0% by weight |
| 6) White Vaseline (Trade Mark) | 67.5% by weight |

Method

A) Heat and melt 3) to 6).

B) Disperse 1) in 2) with heating, and add this solution to the mixture of 3) to 6).

C) Stir the resulting mixture until it has cooled to room temperature and becomes a semi-solid preparation.

Example 3

In this Example and Examples 4 to 9 below, the preparations were produced in the same manner as in Example 1 or Example 2, as appropriate.

| | |
|---|---|
| 1) Indomethacin | 0.5% by weight |
| 2) White Vaseline (Trade Mark) | 15.0% by weight |
| 3) Liquid paraffin | 6.0% by weight |
| 4) Cetanol | 3.0% by weight |
| 5) Stearyl alcohol | 3.0% by weight |
| 6) Polyoxyethylene cetyl ether | 2.5% by weight |
| 7) Propylene glycol | 5.0% by weight |
| 8) Purified water | An amount sufficient to bring the final weight to 100% |

Example 4

| | |
|---|---|
| 1) Indomethacin | 0.5% by weight |
| 2) Propylene glycol | 15.0% by weight |
| 3) Sorbitan sesquioleate | 1.0% by weight |
| 4) Liquid paraffin | 10.0% by weight |
| 5) Solid paraffin | 5.0% by weight |
| 6) White Vaseline (Trade Mark) | 68.5% by weight |

Example 5

| | |
|---|---|
| 1) Indomethacin | 0.5% by weight |
| 2) White Vaseline (Trade Mark) | 15.0% by weight |
| 3) Liquid paraffin | 6.0% by weight |
| 4) Cetanol | 3.0% by weight |
| 5) Stearyl alcohol | 3.0% by weight |
| 6) Polyoxyethylene cetyl ether | 2.5% by weight |
| 7) Crotamiton | 5.0% by weight |
| 8) Purified water | An amount sufficient to bring the final weight to 100% |

Example 6

| | |
|---|---|
| 1) Indomethacin | 0.5% by weight |
| 2) Benzyl alcohol | 8.0% by weight |
| 3) Sorbitan sesquioleate | 1.0% by weight |
| 4) Liquid paraffin | 10.0% by weight |
| 5) Solid paraffin | 5.0% by weight |
| 6) White Vaseline (Trade Mark) | 75.5% by weight |

Example 7

| | |
|---|---|
| 1) Indomethacin | 0.5% by weight |
| 2) Polyoxyethylene sorbitan monooleate | 20.0% by weight |
| 3) Liquid paraffin | 10.0% by weight |
| 4) Solid paraffin | 5.0% by weight |
| 5) White Vaseline (Trade Mark) | 64.5% by weight |

Example 8

| | |
|---|---|
| 1) Indomethacin | 1.0% by weight |
| 2) Stearyl monoglyceride | 10.0% by weight |
| 3) Stearic acid | 2.0% by weight |
| 4) Polyoxypropylene cetyl ether | 3.0% by weight |
| 5) Isopropyl myristate | 20.0% by weight |
| 6) Diethyl sebacate | 5.0% by weight |
| 7) Polyethylene glycol | 10.0% by weight |
| 8) Paraoxymethyl benzoate+ paraoxypropyl benzoate | 0.2% by weight |
| 9) Purified water | An amount sufficient to bring the final weight to 100% |

Example 9

Clinical studies on the remedial effect of the external preparations according to the present invention were conducted in three selected medical institutions. In these studies, the ointment produced in Example 8 was used and evaluated by application to 60 patients suffering from dermatoses.

The results obtained are as shown in Tables 3 and 4.

TABLE 3
Efficacy by dermatoses

| Dermatosis | Degree of efficacy*) | | | | Number of dermatoses |
|---|---|---|---|---|---|
| | A | B | C | D | |
| Herpes zoster | 5 | 18 | 5 | 1 | 29 |
| Herpes simplex | 1 | 2 | 1 | 0 | 4 |
| Eczema and dermatites | 2 | 0 | 0 | 1 | 3 |
| Erythemas | 4 | 7 | 8 | 0 | 19 |
| Scalding | 1 | 0 | 1 | 0 | 2 |
| Chilblain | 2 | 1 | 0 | 0 | 3 |
| Total | 15 (25.0%) | 28 (46.7%) | 15 (25.0%) | 2 (3.3%) | 60 (100%) |

TABLE 4
Efficacy by symptoms

| Symptom | Degree of efficacy*) | | | | Number of symptoms |
|---|---|---|---|---|---|
| | A | B | C | D | |
| Pain | 15 | 17 | 0 | 1 | 33 |
| Erythema | 12 | 16 | 4 | 1 | 33 |
| Swelling | 10 | 7 | 10 | 3 | 30 |
| Total | 37 (38.5%) | 40 (41.7%) | 14 (14.6%) | 5 (5.2%) | 96**) (100%) |

*) The degree of efficacy is expressed as follows:
A: Excellent
B: Fair
C: Good
D: Bad or ineffective
**) Because some of the patients had more than one symptom, the total number of symptoms is greater than that of the dermatoses.

**Claims**

1. An external preparation in the form of an ungelled ointment or cream for the treatment of dermatoses, comprising as essential ingredients, other than are required to convert or maintain the preparation in ungelled ointment or cream form, or conventionally used in such ointments or creams, an effective amount of indomethacin and at least one adjuvant selected from a liquid monohydric alcohol, a polyhydric alcohol, an ester of a monobasic fatty acid and a surface active agent, but not including N,N-diethyl-m-toluamide, wherein said liquid monohydric alcohol is ethanol, isopropanol or benzyl alcohol, said polyhydric alcohol is propylene glycol, said ester of a monobasic fatty acid is isopropyl palmitate, lauryl lactate or decyl oleate, and said surface active agent is polyoxyethylene sorbitan monooleate, a polyoxyethylene hardened castor oil derivative or a polyoxyethylene higher alcohol ether.

2. A preparation as claimed in Claim 1, characterised in that said indomethacin is present in an amount of 0.1 to 7% by weight of the preparation.

3. A preparation as claimed in Claim 1, characterised in that said indomethacin is present in an amount of from 0.5 to 5.0% by weight of the preparation.

4. A preparation as claimed in Claim 1, characterised in that said adjuvant is present in an amount of from 0.1 to 50% by weight of the preparation.

5. A preparation as claimed in any one of claims 1 to 4, for use in the treatment of one of the dermatoses, dermatitis, eczema, psoriasis, zoster, ulcerative dermatitis and decubitus.

**Patentansprüche**

1. Ein äusserliches Präparat in Form einer ungelierten Salbe oder Creme für die Behandlung von Dermatosen, enthaltend als wesentliche Bestandteile, anders als die, welche erforderliche sind, um das Präparat in ungelierte Form zu bringen oder es in dieser zu erhalten, oder welche, wie üblich, in solchen Salben oder Cremen verwendet werden, eine wirksame Menge von Indomethazin und mindestens ein Adjuvans ausgewählt aus einem flüssigen, monohydratischen Alkohol, einem mehrere Hydroxylgruppen enthaltenden Alkohol, einem Ester aus einbasischer Fettsäure und einem oberflächenaktiven Agens, nicht aber einschliessend N,N-Diethyl-m-Toluamid, worin der genannte monohydratische Alkohol Äthanol, Isopropanol oder Benzylalkohol ist, der genannte mehrere Hydroxylgruppen enthaltende Alkohol Propylenglykol ist, der genannte Ester einer einbasischen Fettsäure Isopropylpalmitat, Laurinlaktat oder Decyloleinat ist, und das genannte aktive Oberflächenagens Polyoxyethylensorbitanmonooleinat, ein Polyoxyethylen-gehärtetes Rizinusöl-Derivat oder ein Polyoxyethylen-höherer Alkoholäther ist.

2. Ein Präparat gemäss Anspruch 1, dadurch gekennzeichnet, dass das genannte Indomethazin in einer Menge von 0,1 bis 7% des Gewichts des Präparats vorhanden ist.

3. Ein Präparat gemäss Anspruch 1, dadurch gekennzeichnet, dass das genannte Indomethazin in einer Menge von 0,5 bis 5,0% des Gewichts des Präparats vorhanden ist.

4. Ein Präparat gemäss Anspruch 1, dadurch gekennzeichnet, dass das genannte Adjuvans in einer Menge von 0,1 bis 50% des Gewichts des Präparats vorhanden ist.

5. Ein Präparat gemäss einem jeden der Ansprüche 1 bis 4 für die Behandlung von Dermatose, Dermatitis, Ekzem, Psoriasis, Herpes, geschwürartiger Dermatitis oder Dekubitus.

**Revendications**

1. Préparation à usage externe, ayant la forme d'un onguent non gélifié ou d'une crème pour le traitement des dermatoses, comprenant, comme ingrédients essentiels autres que ceux nécessaires à convertir ou maintenir la préparation sous forme d'onguent non gélifié ou de crème, ou que ceux utilisés traditionnellement dans ces onguents ou crèmes, un proportion active d'indométhacine et au moins un adjuvant sélectionné parmi les monoalcools monovalents liquides, les polyalcools, les esters d'un acide gras monobasique et les agents tensio-actifs, mais en excluant le N,N-diéthyl-m-toluamide, le monoalcool liquide étant l'éthanol, l'isopropanol ou l'alcool benzyliquie, le polyalcool étant le propylene glycol, l'ester d'acide gras monobasique étant le palmitate d'isopropyle, le lactate de lauryle ou l'oléate de décyle, et l'agent tensioactif étant le monooléate de sorbitan polyoxyéthyléné, un dérivé de l'huile de ricin durci polyoxyéthylené ou éther d'alcool supérieur polyoxyéthyléné.

2. Préparation selon la revendication 1, caractérisée en ce que l'indométhacine est présente dans une proportion de 0,1 à 7% en poids de la préparation.

3. Préparation selon la revendication 1, caractérisée en ce que l'indométhacine est présente dans une proportion de 0,5 a 5% en poids de la préparation.

4. Préparation selon la revendication 1, caractérisée en ce que l'adjuvant est present dans une proportion de 0,1 a 50% en poids de la préparation.

5. Préparation selon l'une quelconque des revendications 1 à 4, destinée à l'utilisation dans le traitement des dermatoses, dermatites, eczémas, psoriasis, zonas, dermatites à ulcères et décubitus.

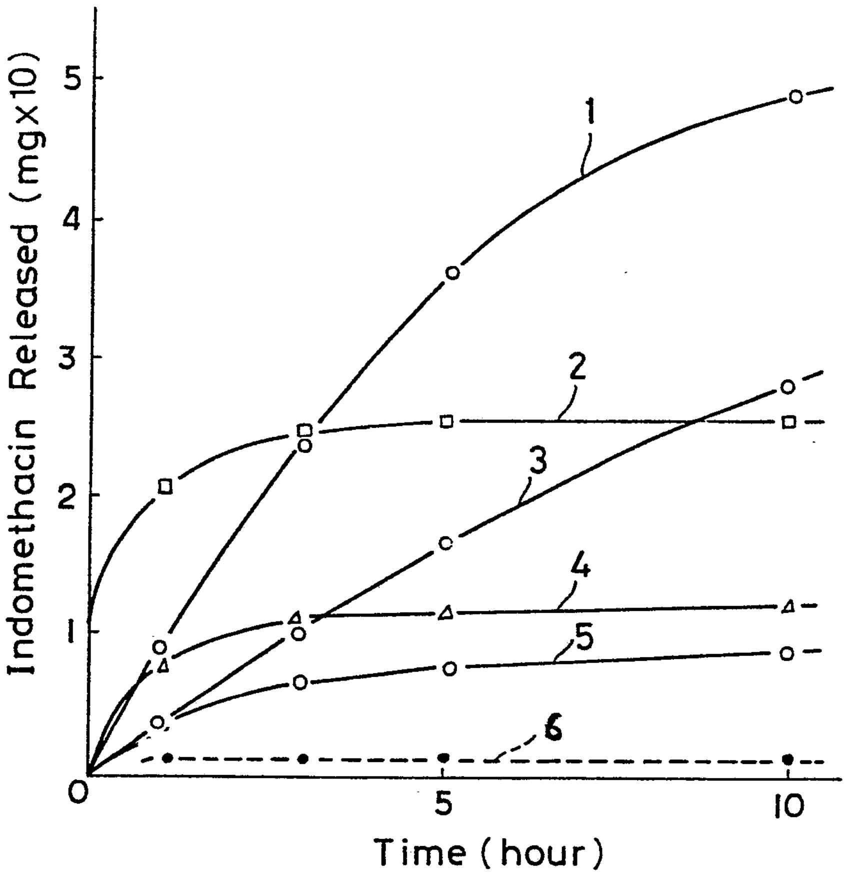
Indomethacin Released (mg×10)
5
4
3
2
1
0
5
10
Time (hour)
1
2
3
4
5
6